# FASCICULE DE BREVET EUROPEEN

(11) **EP 2 016 051 B1**
(45) Date de publication et mention de la délivrance du brevet: **28.04.2010**
(21) Numéro de dépôt: 07731038.1
(22) Date de dépôt: 26.02.2007
(51) Int. Cl.: C07D 209/42, A61K 31/404

(54) **FORME CRISTALLINE BETA DU SEL D'ARGININE DU PERINDOPRIL, SON PROCEDE DE PREPARATION, ET LES COMPOSITIONS PHARMACEUTIQUES QUI LA CONTIENNENT**
BETA-KRISTALLINE FORM VON PERINDOPRIL-ARGININ-SALZ, VERFAHREN ZU DESSEN HERSTELLUNG UND DIESES ENTHALTENDE PHARMAZEUTISCHE ZUBEREITUNGEN
BETA-CRYSTALLINE FORM OF PERINDOPRIL ARGININE SALT, PROCESS FOR ITS PREPARATION, AND PHARMACEUTICAL COMPOSITIONS CONTAINING IT

(30) Priorité: 28.02.2006 FR 0601747
(43) Date de publication de la demande: 21.01.2009
(73) Titulaire: Les Laboratoires Servier, 92415 Courbevoie Cedex (FR)
(72) Inventeur: COQUEREL, Gérard, F-76520 Boos (FR); LEFEBVRE, Loïc, 76130 Mont Saint Aignan (FR); SOUVIE, Jean-Claude, F-64000 PAU (FR); AUTHOUART, Pascale, F-76430 Les Trois Pierres (FR)
(74) Mandataire: Giudicelli, Cathy
(86) Numéro de dépôt international: PCT/FR2007/000334
(87) Numéro de publication internationale: WO 2007/099216

(56) Documents cités:
- EP-A1- 1 354 873
- WO-A-01/87835
- WO-A-01/87836
- WO-A-2004/000286
- WO-A2-01/83439

## Description

La présente invention concerne la forme cristalline β du sel de L-arginine du perindopril de formule (I) : 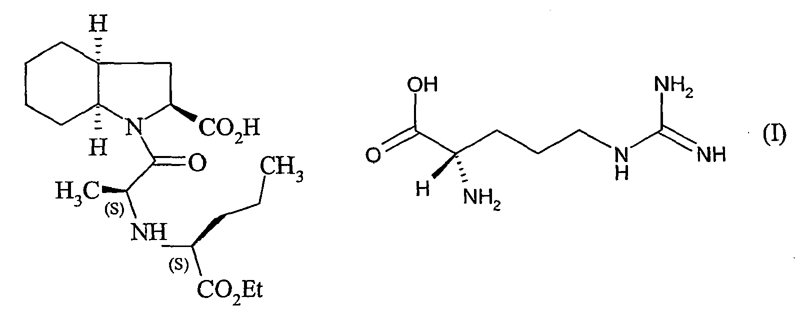 son procédé de préparation ainsi que les compositions pharmaceutiques qui la contiennent.

Le perindopril, ainsi que ses sels pharmaceutiquement acceptables, et plus particulièrement son sel d'arginine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Le sel d'arginine du perindopril a été décrit dans le brevet européen EP 1 354 873.

Compte tenu de l'intérêt pharmaceutique de ce composé, il était primordial de l'obtenir avec une excellente stabilité, principalement en termes d'hygroscopicité, de processabilité de la poudre, de filtrabilité du solide, de broyage, de rétention de solvant.

L'obtention d'une forme cristalline bien définie permet de répondre à ce cahier des charges.

Le brevet EP 1 354 873 décrit le sel d'arginine du perindopril. Cependant, ce document ne précise pas les conditions d'obtention de ce sel sous une forme cristalline bien définie.

La demanderesse a présentement trouvé que le sel d'arginine du perindopril pouvait être obtenu sous une forme cristalline bien définie, présentant de ce fait des caractéristiques intéressantes de filtration, de séchage et de facilité de formulation.

Plus spécifiquement, la présente invention concerne la forme cristalline β du composé de formule (I), caractérisée par les pics de diffraction RX sur poudre suivants, mesurés sur un diffractomètre à anticathode de cuivre et exprimés en termes d'angle 2-thêta (°) : 4,3, 19,1 et 21,6.

De façon préférentielle, la présente invention concerne la forme cristalline β du composé de formule (I), caractérisée par les pics de diffraction RX sur poudre suivants, mesurés sur un diffractomètre à anticathode de cuivre et exprimés en termes d'angle 2-thêta (°) : 4,3, 11,1, 12,2, 16,2, 19,1 19,6 et 21,6.

De façon encore plus préférentielle, la présente invention concerne la forme cristalline β du composé de formule (I), caractérisée par le diagramme de diffraction X sur poudre suivant, mesuré sur un diffractomètre (anticathode de cuivre) et exprimé en termes de distance inter-réticulaire d, d'angle de Bragg 2 thêta, d'intensité et d'intensité relative (exprimée en pourcentage par rapport à la raie la plus intense) :

| **Angle 2 thêta (°)** | **Distance inter-réticulaire d (Å)** | **Intensité** | **Intensité relative (%)** |
|---|---|---|---|
| *4,29* | *20,58* | *2314* | *100,0* |
| 9,73 | 9,088 | 257 | 11,1 |
| 11,083 | 7,977 | 735 | 31,8 |
| 11,400 | 7,756 | 452 | 19,5 |
| 12,237 | 7,227 | 762 | 32,9 |
| 13,050 | 6,778 | 657 | 28,4 |
| 14,604 | 6,061 | 619 | 26,8 |
| 15,039 | 5,886 | 436 | 18,8 |
| 16,188 | 5,471 | 1025 | 44,3 |
| 19,168 | 4,627 | 1820 | 78,6 |
| 19,622 | 4,521 | 1016 | 43,9 |
| 21,666 | 4,098 | 1352 | 58,4 |
| 22,892 | 3,882 | 430 | 18,6 |
| 23,399 | 3,799 | 394 | 17,0 |
| 24,197 | 3,675 | 232 | 10,0 |
| 26,024 | 3,421 | 298 | 12,9 |
| 29,490 | 3,026 | 291 | 12,6 |

L'invention s'étend également au procédé de préparation de la forme cristalline β du composé de formule (I), dans lequel le sel de (L)-arginine du perindopril est chauffé au reflux du toluène ou de l'acétonitrile, puis les cristaux obtenus sont filtrés à chaud et séchés.

L'invention s'étend aussi aux compositions pharmaceutiques renfermant comme principe actif la forme cristalline β du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les compositions pharmaceutiques selon l'invention peuvent également contenir un diurétique comme l'indapamide.

Les exemples suivants illustrent l'invention.

Le spectre de diffraction X sur poudre a été mesuré avec les conditions expérimentales suivantes :
Diffractomètre Siemens D5005 ; détecteur à scintillations ;
Anticathode cuivre, voltage 40KV, intensité 30mA ;
Montage θ - θ, échantillon fixe ;
Température : ambiante ;
Domaine de mesures : 3° à 30° ;
Incrémentation entre chaque mesure : 0,04° ;
Temps de mesure par pas : 4s ;
Fentes fixes : 1,6mm ;
Filtre Kβ (Ni) ;
Pas de référence interne ;
Procédure de zéro avec les fentes Siemens ;
Données expérimentales traitées avec le logiciel EVA (version 9.0).

### EXEMPLE 1 : Forme cristalline β du sel d'arginine du perindopril

Dans un réacteur sont introduits sous agitation 100 g de sel de (L)-arginine du perindopril et 5 1 d'acétonitrile. Le mélange est ensuite porté au reflux sous agitation. Le solide, initialement en suspension, se solubilise totalement, puis précipite à chaud après quelques minutes. Après 1h d'agitation à reflux, les cristaux obtenus sont filtrés à une température de 80°C. Les cristaux obtenus sont séchés, pour conduire au produit du titre sous forme anhydre.

### Diagramme de diffraction X sur poudre :

Le profil de diffraction des rayons X de la poudre (angles de diffraction) de la forme β du sel d'arginine du perindopril est donné par les raies significatives rassemblées dans le tableau suivant, avec l'intensité et l'intensité relative (exprimée en pourcentage par rapport à la raie la plus intense).

| **Angle 2 thêta (°)** | **Distance inter-réticulaire d (Å)** | **Intensité** | **Intensité relative (%)** |
|---|---|---|---|
| *4,29* | *20,58* | *2314* | *100,0* |
| 9,73 | 9,088 | 257 | 11,1 |
| 11,083 | 7,977 | 735 | 31,8 |
| 11,400 | 7,756 | 452 | 19,5 |
| 12,237 | 7,227 | 762 | 32,9 |
| 13,050 | 6,778 | 657 | 28,4 |
| 14,604 | 6,061 | 619 | 26,8 |
| 15,039 | 5,886 | 436 | 18,8 |
| 16,188 | 5,471 | 1025 | 44,3 |
| 19,168 | 4,627 | 1820 | 78,6 |
| 19,622 | 4,521 | 1016 | 43,9 |
| 21,666 | 4,098 | 1352 | 58,4 |
| 22,892 | 3,882 | 430 | 18,6 |
| 23,399 | 3,799 | 394 | 17,0 |
| 24,197 | 3,675 | 232 | 10,0 |
| 26,024 | 3,421 | 298 | 12,9 |
| 29,490 | 3,026 | 291 | 12,6 |

### EXEMPLE 2 : Composition pharmaceutique

Formule de préparation pour 1000 comprimés dosés à 4 mg :

| | |
|---|---|
| Composé de l'exemple 1 | 4 g |
| Hydroxypropylcellulose | 2 g |
| Amidon de blé | 10 g |
| Lactose | 100 g |
| Stéarate de magnésium | 3 g |
| Talc | 3 g |

## Revendications

1. Forme cristalline β du sel de L-arginine du perindopril, de formule (I) : 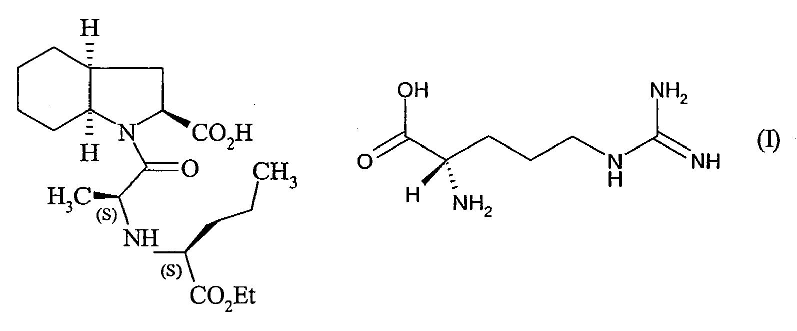 **caractérisée par** les pics de diffraction RX sur poudre suivants, mesurés sur un diffractomètre à anticathode de cuivre et exprimés en termes d'angle de Bragg 2-thêta (°) : 4,3, 19,1 et 21,6.

2. Forme cristalline β du composé de formule (I) selon la revendication 1, **caractérisée par** les pics de diffraction RX sur poudre suivants, mesurés sur un diffractomètre à anticathode de cuivre et exprimés en termes d'angle de Bragg 2-thêta (°) :4,3, 11,1, 12,2, 16,2, 19,1 19,6 et 21,6.

3. Forme cristalline β du composé de formule (I) selon la revendication 1, **caractérisée par** le diagramme de diffraction X sur poudre suivant, mesuré sur un diffractomètre à anticathode de cuivre et exprimé en termes de distances inter-réticulaires d, d'angle de Bragg 2 thêta, d'intensité et d'intensité relative exprimée en pourcentage par rapport à la raie la plus intense:
| **Angle 2 thêta (°)** | **Distace inter-réticulaire d (Å)** | **Intensité** | **Intensité relative: (%)** |
|---|---|---|---|
| *4,29* | *20,58* | *2314* | *100,0* |
| 9,73 | 9,088 | 257 | 11,1 |
| 11,083 | 7,977 | 735 | 31,8 |
| 11,400 | 7,756 | 452 | 19,5 |
| 12,237 | 7,227 | 762 | 32,9 |
| 13,050 | 6,778 | 657 | 28,4 |
| 14,604 | 6,061 | 619 | 26,8 |
| 15,039 | 5,886 | 436 | 18,8 |
| 16,188 | 5,471 | 1025 | 44,3 |
| 19,168 | 4,627 | 1820 | 78,6 |
| 19,622 | 4,521 | 1016 | 43,9 |
| 21,666 | 4,098 | 1352 | 58,4 |
| 22,892 | 3,882 | 430 | 18,6 |
| 23,399 | 3,799 | 394 | 17,0 |
| 24,197 | 3,675 | 232 | 10,0 |
| 26,024 | 3,421 | 298 | 12,9 |
| 29,490 | 3,026 | 291 | 12,6 |

4. Procédé de préparation de la forme cristalline β du composé de formule (I) selon l'une quelconque des revendications 1 à 3, dans lequel le sel de (L)-arginine du perindopril est chauffé au reflux du toluène ou de l'acétonitrile, puis les cristaux obtenus sont filtrés à chaud et séchés.

5. Composition pharmaceutique contenant comme principe actif le composé selon l'une quelconque des revendications 1 à 3, en combinaison avec un ou plusieurs véhicules inertes, non toxiques et pharmaceutiquement acceptables.

6. Composition pharmaceutique selon la revendication 5, **caractérisée en ce qu'**elle contient également un diurétique.

7. Composition pharmaceutique selon la revendication 6, **caractérisée en ce que** le diurétique est l'indapamide.

8. Utilisation du composé selon l'une quelconque des revendications 1 à 3 pour la fabrication de médicaments utiles en tant qu'inhibiteur de l'enzyme de conversion de l'angiotensine I.

9. Utilisation du composé selon l'une quelconque des revendications 1 à 3 pour la fabrication de médicaments utiles dans le traitement des maladies cardiovasculaires.

## Claims

1. β crystalline form of the L-arginine salt of perindopril, of formula (I) : 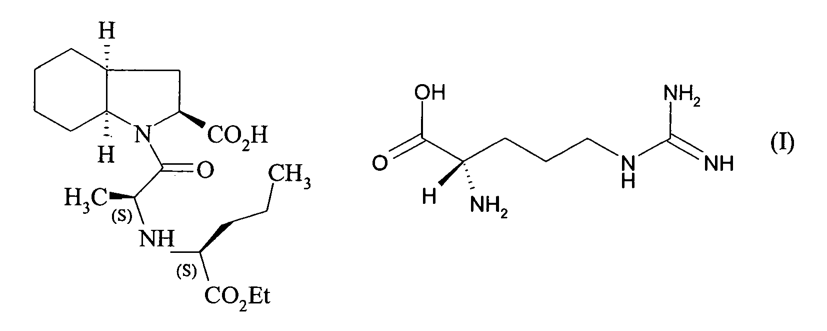 **characterised by** the following X-ray powder diffraction peaks, measured using a diffractometer with a copper anti-cathode and expressed in terms of Bragg's angle 2 theta (°) : 4.3, 19.1 and 21.6.

2. β crystalline form of the compound of formula (I) according to claim 1, **characterised by** the following X-ray powder diffraction peaks, measured using a diffractometer with a copper anti-cathode and expressed in terms of Bragg's angle 2 theta (°) : 4.3, 11.1, 12.2, 16.2, 19.1, 19.6 and 21.6.

3. β crystalline form of the compound of formula (I) according to claim 1, **characterised by** the following X-ray powder diffraction diagram measured using a diffractometer with a copper anti-cathode and expressed in terms of inter-planar distance d, Bragg's angle 2 theta, intensity and, expressed as a percentage of the most intense line, relative intensity :
| **Angle 2 theta (°)** | **Inter-planar distance d (Å)** | **Intensity** | **Relative intensity (%)** |
|---|---|---|---|
| *4.29* | *20.58* | *2314* | *100.0* |
| 9.73 | 9.088 | 257 | 11.1 |
| 11.083 | 7.977 | 735 | 31.8 |
| 11.400 | 7.756 | 452 | 19.5 |
| 12.237 | 7.227 | 762 | 32.9 |
| 13.050 | 6.778 | 657 | 28.4 |
| 14.604 | 6.061 | 619 | 26.8 |
| 15.039 | 5.886 | 436 | 18.8 |
| 16.188 | 5.471 | 1025 | 44.3 |
| 19.168 | 4.627 | 1820 | 78.6 |
| 19.622 | 4.521 | 1016 | 43.9 |
| 21.666 | 4.098 | 1352 | 58.4 |
| 22.892 | 3.882 | 430 | 18.6 |
| 23.399 | 3.799 | 394 | 17.0 |
| 24.197 | 3.675 | 232 | 10.0 |
| 26.024 | 3.421 | 298 | 12.9 |
| 29.490 | 3.026 | 291 | 12.6 |

4. Process for the preparation of the P crystalline form of the compound of formula (I) according to any one of claims 1 to 3, wherein the (L)-arginine salt of perindopril is heated with toluene or acetonitrile, at reflux, and then the crystals obtained are filtered off in the hot state and dried.

5. Pharmaceutical composition comprising as active ingredient the compound according to any one of claims 1 to 3, in combination with one or more pharmaceutically acceptable, inert, non-toxic carriers.

6. Pharmaceutical composition according to claim 5, **characterised in that** it also contains a diuretic.

7. Pharmaceutical composition according to claim 6, **characterised in that** the diuretic is indapamide.

8. Use of the compound according to any one of claims 1 to 3 in the manufacture of medicaments for use as inhibitors of angiotensin I converting enzyme.

9. Use of the compound according to any one of claims 1 to 3 in the manufacture of medicaments for use in the treatment of cardiovascular diseases.

## Patentansprüche

1. β-Kristallform des L-Argininsalzes von Perindopril der Formel (I): 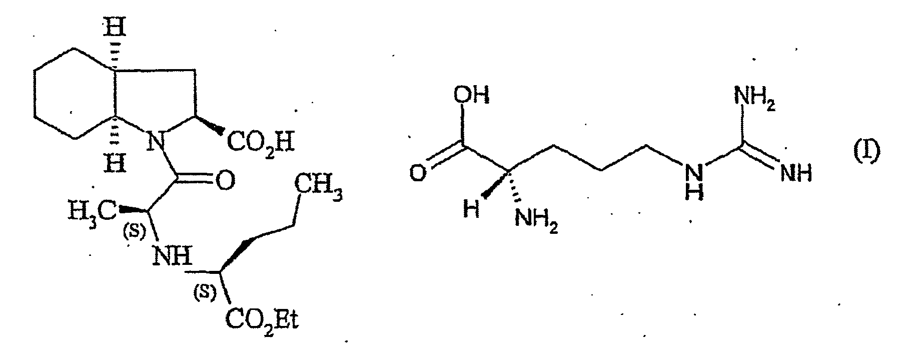 **gekennzeichnet durch** die folgenden Pulverröngtenbeugungspeaks, gemessen mit einem Diffraktometer mit Kupfer-Antikathode und angegeben als Bragg-Winkel 2-Theta (°) : 4,3, 19,1 und 21,6.

2. β-Kristallform der Verbindung der Formel (I) nach Anspruch 1, **gekennzeichnet durch** die folgenden Pulverröntgenbeugungspeaks, gemessen mit einem Diffraktometer mit Kupfer-Antikathode und angegeben als Bragg-Winkel 2-Theta (°): 4,3, 11,1, 12,2, 16,2, 19,1, 19,6 und 21,6.

3. β-Kristallform der Verbindung der Formel (I) nach Anspruch 1, **gekennzeichnet durch** das folgende Pulverröntgenbeugungsdiagramm, gemessen mit einem Diffraktometer mit Kupfer-Antikathode und angegeben **durch** die Schichtabstände d, die Bragg-Winkel 2-Theta, die Intensität und die relative Intensität angegeben als Prozentsatz bezogen auf die intensivste Bande:
| **2-Theta-Winkel (°)** | **Gitterabstand d (Å)** | **Intensität** | **Relative Intensität (%)** |
|---|---|---|---|
| *4,29* | *20,58* | *2314* | *100,0* |
| 9,73 | 9,088 | 257 | 11,1 |
| 11,083 | 7,977 | 735 | 31,8 |
| 11,400 | 7,756 | 452 | 19,5 |
| 12,237 | 7,227 | 762 | 32,9 |
| 13,050 | 6,778 | 657 | 28,4 |
| 14,604 | 6,061 | 619 | 26,8 |
| 15,039 | 5,886 | 436 | 18,8 |
| 16,188 | 5,471 | 1025 | 44,3 |
| 19,168 | 4,627 | 1820 | 78,6 |
| 19,622 | 4,521 | 1016 | 43,9 |
| 21,666 | 4,098 | 1352 | 58,4 |
| 22,892 | 3,882 | 430 | 18,6 |
| 23,399 | 3,799 | 394 | 17,0 |
| 24,197 | 3,675 | 232 | 10,0 |
| 26,024 | 3,421 | 298 | 12,9 |
| 29,490 | 3,026 | 291 | 12,6 |

4. Verfahren zur Herstellung der β-Kristallform der Verbindung der Formel (I) nach einem der Ansprüche 1 bis 3, worin das (L)-Argininsalz von Perindopril in Toluol oder Acetonitril zum Rückfluss erhitzt wird, dann die erhaltenen Kristalle in der Wärme abfiltriert und getrocknet werden.

5. Pharmazeutische Zubereitung enthaltend als Wirkstoff die Verbindung nach einem der Ansprüche 1 bis 3 in Kombination mit einem oder mehreren inerten, nichttoxischen und pharmazeutisch annehmbaren Hilfsstoffen.

6. Pharmazeutische Zubereitung nach Anspruch 5, **dadurch gekennzeichnet, dass** sie zusätzlich ein Diuretikum enthält.

7. Pharmazeutische Zubereitung nach Anspruch 6, **dadurch gekennzeichnet, dass** das Diuretikum Indapamid ist.

8. Verwendung der Verbindung nach einem der Ansprüche 1 bis 3 für die Herstellung von Arzneimitteln, die nützlich sind als Inhibitoren des Angiotensin I umwandelnden Enzyms.

9. Verwendung der Verbindung nach einem der Ansprüche 1 bis 3 für die Herstellung von Arzneimitteln, die nützlich sind bei der Behandlung von kardiovaskulären Erkrankungen.
